(19) Europäisches Patentamt
European Patent Office
Office européen des brevets

(11) Veröffentlichungsnummer: **0 100 527**
**B1**

(12) # EUROPÄISCHE PATENTSCHRIFT

(45) Veröffentlichungstag der Patentschrift: **07.01.87**

(51) Int. Cl.⁴: **C 07 D 279/16**, A 61 K 31/54

(21) Anmeldenummer: **83107419.0**

(22) Anmeldetag: **28.07.83**

(54) **4H-1,4-Benzothiazine zur Verwendung als Arzneimittel.**

(30) Priorität: **04.08.82 DE 3229121**

(43) Veröffentlichungstag der Anmeldung:
**15.02.84 Patentblatt 84/7**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

(56) Entgegenhaltungen:
**EP - A - 0 055 917**
**EP - A - 0 061 082**

**Immunology 40, 709 (1980)**
**Medical Clinics of North America 65, 809 (1981)**
**Liebigs Annalen der Chemie 744, 27 (1971)**

***Die Akte enthält technische Angaben, die nach dem Eingang der Anmeldung eingereicht wurden und die nicht in dieser Patentschrift enthalten sind.***

(73) Patentinhaber: **BAYER AG, Konzernverwaltung RP Patentabteilung, D-5090 Leverkusen 1 Bayerwerk (DE)**

(72) Erfinder: **Niemers, Ekkehard, Dr., In den Birken 51a, D-5600 Wuppertal 1 (DE)**
Erfinder: **Grützmann, Rudi, Dr., Helsinkistrasse 20, D-5650 Solingen 1 (DE)**
Erfinder: **Mardin, Mithat, Dr., Untere Bergerheide 15, D-5600 Wuppertal 1 (DE)**
Erfinder: **Busse, Wolf-Dieter, Dr., 400 Morgan Lane, West Haven, Ct 06516 (US)**
Erfinder: **Meyer, Horst, Dr., Henselweg 11, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 100 527 B1

**Beschreibung**

Die vorliegende Erfindung betrifft 4H-1,4-Benzothiazine zur therapeutischen Verwendung bzw. zur Verwendung als Arzneimittel, insbesondere ihre Verwendung als Lipoxygenasehemmer, ferner diese enthaltende Arzneimittel und deren Herstellung.

Es ist bekannt, dass die durch das Enzym Lipoxygenase gebildeten Metaboliten der Arachidonsäure-Leukotriene und slow reacting Substance of anaphylaxis (SRS-A) an der Entstehung von entzündlichen und allergischen Prozessen beteiligt sind (E.J. Goetzl, Immunology 40, 709 (1980) und Medical Clinics of North America 65, 809 (1981)).

Bekannte Lipoxygenasehemmer wie Nordihydroguaretsäure, 3-Amino-1-(3-trifluormethylphenyl)-pyrazolin, Phenindione und 5,8,11,14-Eikosatetrainsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und zu einer Stimulation des Lipoxygenaseweges, was eine Gastrotoxizität bzw. pro-inflammatorische und asthmatische Wirkungen verursacht. Ausserdem haben schon bekannte Lipoxygenasehemmer wie 3-Amino-1-(m-trifluormethylphenyl)-pyrazolin-2 bei systemischer Verabreichung (z.B. oral) toxische Nebenwirkungen. Es besteht deshalb ein Bedarf nach oral wirksamen Verbindungen, die diese unerwünschten Nebenwirkungen nicht besitzen.

Aus der EP 055 917 sind Benzothiazine bekannt, die bei Erkrankungen des Autoimmunsystems verwendet werden können.

In der EP 0 061 082 wird ein Verfahren zur Herstellung von Benzothiazinen beschrieben, die als Zwischenprodukte verwendet werden können.

In Liebigs Annalen der Chemie 744, 27 bis 32 (1971) werden Verfahren zur Herstellung von Benzothiazinen beschrieben.

Es wurden 4H-1,4-Benzothiazine der Formel

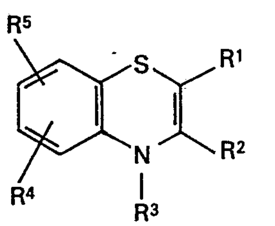

(I),

in der

$R^1$ Wasserstoff, Aryl mit 6 bis 20 Kohlenstoffatomen, das gegebenenfalls ein- bis dreimal durch Halogen, Trifluormethyl oder Nitro substituiert sein kann, 2-Thienyl, 3-Thienyl, 2-Furyl, 3-Furyl, 2-Pyridyl,

3-Pyridyl, 4-Pyridyl, 2-Pyrimidyl, 4-Pyrimidyl oder 5-Pyrimidyl, die gegebenenfalls durch Alkyl, Alkoxy, Hydroxy oder Halogen ein- oder mehrfach substituiert sein können, Cyano oder die Gruppe

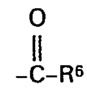

bedeutet, in der

$R^6$ für Alkyl ($C_1$ bis $C_8$), Aryl ($C_6$ bis $C_{20}$), Amino, Pyrrolidino, Piperidino, Morpholino, Cycloalkoxy oder für den Rest

$- O - CH_2 - R^8$

steht, in dem

$R^8$ geradkettiges oder verzweigtes Alkyl mit 1 bis 7 Kohlenstoffatomen, das gegebenenfalls ein- bis dreimal durch Halogen, $C_6$ bis $C_{20}$-Arxl, Hydroxy, Alkoxy, Alkylthio oder Cyano substituiert ist, bedeutet,

$R^2$ Alkyl ($C_1$ bis $C_8$) bedeutet, dass gegebenenfalls ein- bis dreimal durch Halogen, Pyrrolidino, Piperidino, Aryl ($C_1$ bis $C_{20}$) oder durch die Gruppe

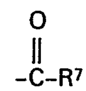

in der

$R^7$ für Aryl ($C_6$ bis $C_{20}$), Hydroxy, Amino, Dialkylamino, Pyrrolidino, Piperidino oder Morpholino steht, substituiert ist,

$R^3$ Wasserstoff oder eine der unter $R^2$ genannten Gruppen bedeutet, und

$R^4$ und $R^5$ gleich oder verschieden sind und Wasserstoff, Halogen, Hydroxy, Alkoxy, Nitro, Cyano, oder eine der unter $R^2$ genannten Gruppen bedeuten, zur therapeutischen Behandlung gefunden.

Überraschenderweise hemmen die genannten 4H-1,4-Benzothiazine die Lipoxygenase bereits in solcher Konzentration, bei denen die Cyclooxygenase wenig beeinflusst wird.

Die genannten Verbindungen stimulieren überraschenderweise auch die Synthese von Prostacyclin in arteriellen Gefässen in vitro, möglicherweise als Folge ihrer lipoxygenasehemmenden Eigenschaft. Bezüglich dieser Wirkung sind die erfindungsgemässen Verbindungen stärker wirksam als der bekannte Lipoxygenasehemmer 3-Amino-1-(m-trifluor-methylphenyl)-pyrazolin-2 (Proc. of British Pharmacological Society 920 P (1981). Die erfindungsgemässen Verbindungen stimulieren auch an Kaninchenaortastreifen die Prostacyclin-Synthese.

Die genannten Verbindungen besitzen auch eine antiphlogistische Wirkung im Carragenaninduzierten Ödemmodell, wenn sie systemisch, besonders oral, und lokal, besonders cutan, verabreicht werden. Sie besitzen ferner antimetastatische Wirkung.

Die genannten lipoxygenasehemmenden 4H-1,4-Benzothiazine sind somit erfindungsgemäss als Arzneimittel bei der Behandlung von entzündlichen und allergischen Prozessen einsetzbar. Sie können insbesondere als Antiphlogistika, Antirheumatika, Antiatherosklerotika, Antithrombotika, Antiarthroika, Antiasthmatika, Antiallergika, Antimetastatika und Gastroprotektiva Verwendung finden.

Es werden erfindungsgemäss zur therapeutischen Verwendung 1,4-Benzothiazine der allgemeinen Formel I bevorzugt, worin

$R^1$ Wasserstoff, Phenyl, Naphthyl, gegebenenfalls ein- oder mehrfach durch Fluor und/oder Chlor substituiertes 2-Thienyl, 3-Thienyl, 2-Furyl, 3-Furyl, 2-Pyridyl, 3-Pyridyl, 4-Pyridyl, 2-Pyrimidyl, 4-Pyrimidyl oder 5-Pyrimidyl, ferner Cyano oder die Gruppe

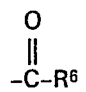

bedeutet, in der

$R^6$ für Alkyl ($C_1$ bis $C_4$), Phenyl, Naphthyl, Amino, Pyrrolidino, Piperidino oder Morpholino oder für den Rest

$-O-CH_2-R^8$

steht, in dem

$R^8$ geradkettiges oder verzweigtes Alkyl mit 1 bis 7 Kohlenstoffatomen bedeutet,

$R^2$ Alkyl ($C_1$ bis $C_4$), das gegebenenfalls durch Fluor und/oder Chlor substituiert ist, bedeutet,

$R^3$ Wasserstoff oder eine der unter $R^2$ genannten Gruppen bedeutet, und

$R^4$ und $R^5$ gleich oder verschieden sind und Wasserstoff, Halogen, Hydroxy, Cyano oder eine der unter $R^2$ genannten Gruppen bedeuten.

Es wurden ausserdem noch 4H-1,4-Benzothiazine der Formeln

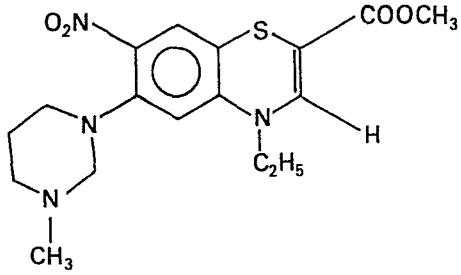

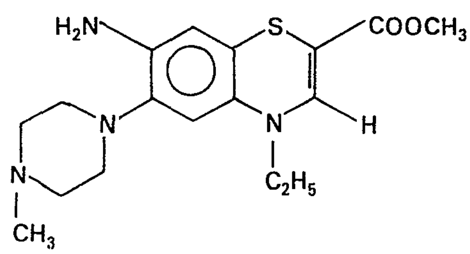

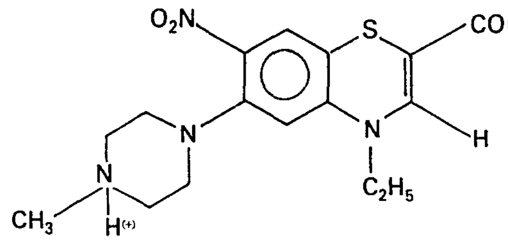

$\times H_2$

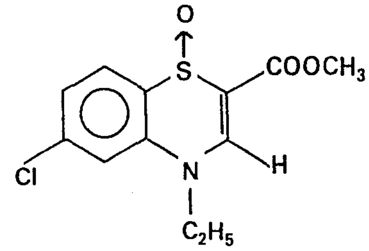

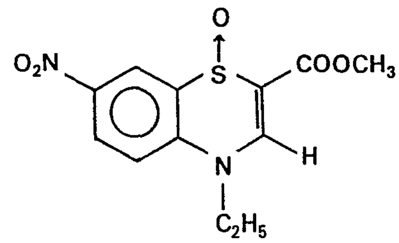

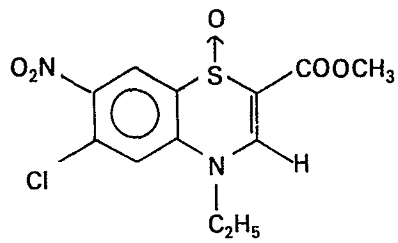

zur therapeutischen Verwendung gefunden.

Die Stoffe der allgemeinen Formel I lassen sich gemäss der Verfahrensvariante 1 herstellen, indem man 2-Aminothiophenole der Formel II mit Verbindungen der Formel III in Dimethylsulfoxid zur Reaktion bringt (vgl. J. Chem. Soc. Perkin Trans. I, 1976, 1146–49).

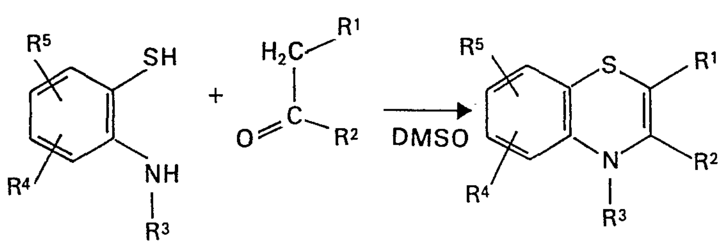


II III I

In den allgemeinen Formeln II und III stehen die Reste $R^1$ bis $R^5$ für die oben angegebenen Bedeutungen.

Die Verbindungen der Formeln II und III sind entweder literaturbekannt oder lassen sich in Analogie zu literaturbekannten Verbindungen herstellen. Die Reaktionstemperatur kann zwischen 50°C und 200°C liegen, vorzugsweise zwischen 100°C und 150°C. In einigen Fällen ist es zweckmässig unter Sauerstoffausschluss zu arbeiten.

Die Stoffe der Formel I lassen sich nach Verfahrensvariante 2 herstellen, indem man 2-Aminothiophenole der Formel II mit Verbindungen der Formel IV zur Reaktion bringt (vgl. Ann. Chem. 744, 20-32 (1971)).

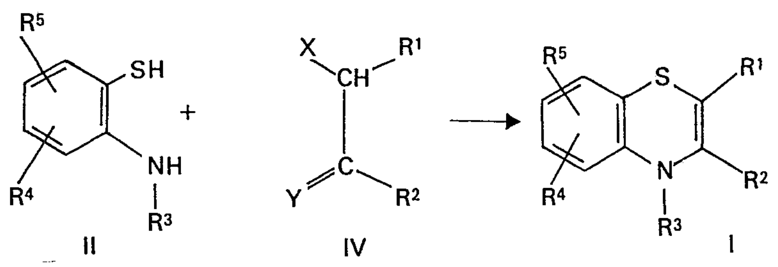

II IV I

X = Halogen, vorzugsweise Cl, Br
Y = Sauerstoff; Alkyl-N; Aryl-N.

Die Reaktion kann vorzugsweise auch in Gegenwart eines Säurefängers, wie z.B. Triethylamin, $K_2CO_3$, $Na_2CO_3$, NaOH, $NH_3$ durchgeführt werden.

Als Lösungsmittel kommen in Frage: Alkohole (z.B. Methanol, Ethanol), Ether (z.B. Diethylether, THF, Dioxan, Dimethoxyethan, Diethylenglykoldimethylether), Kohlenwasserstoffe (z.B. Benzol, Toluol), chlorierte Kohlenwasserstoffe (z.B. Methylenchlorid, Chloroform, Chlorbenzol), DMSO, DMF, Pyridin und Wasser. In einigen Fällen ist es zweckmässig unter Sauerstoffausschluss zu arbeiten.

Die Reaktionstemperatur kann zwischen 0°C und 200°C liegen, vorzugsweise zwischen 35°C und 110°C.

Die Stoffe der Formel I lassen sich nach Verfahrensvariante 3 herstellen, indem man Disulfide der Formel V mit Carbonylverbindungen der Formel III zur Reaktion bringt [vergl. Phosphorus and Sulfur 3, 308-314 (1977)]

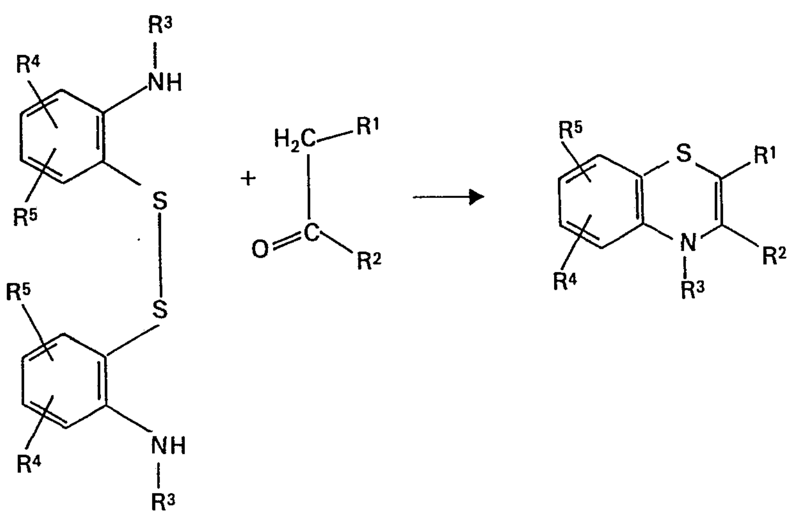

V III I

Die Reaktion wird durch Säuren (z.B. p-Toluolsulfonsäure) katalysiert.

Als Lösungsmittel kommen in Frage: Benzol, Toluol, Pyridin, DMSO und DMF.

Die Reaktionstemperatur kann zwischen 20°C und 180°C liegen, vorzugsweise zwischen 35°C und 110°C.

Es ist zweckmässig unter Sauerstoffausschluss zu arbeiten.

Die Reaktion wird meist mit äquimolekularen Mengen der Ausgangsmaterialien durchgeführt, gelegentlich wird auch ein Überschuss (bis zu 110%) an Carbonylverbindung verwendet.

Die Stoffe der Formel I lassen sich nach Verfahrensvariante 4 herstellen, indem man Disulfide der Formel V mit Alkinen der Formel VI zur Reaktion bringt [vgl. J. Heterocyclic Chem. 17, 377 (1980)].

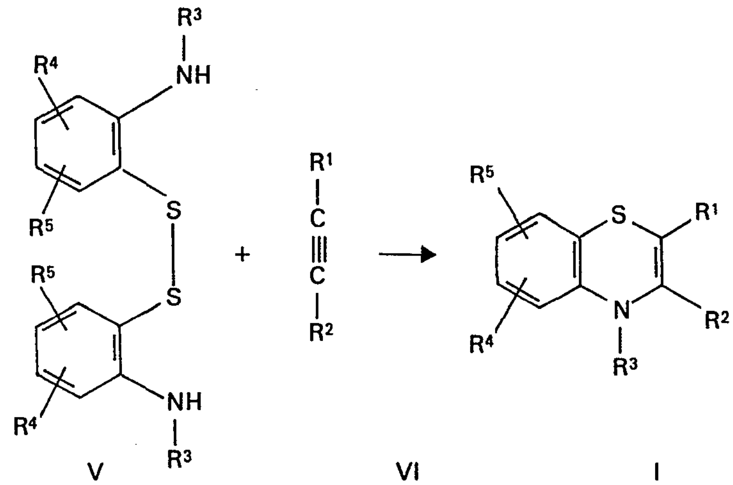

V VI I

Die Reaktion wird vorzugsweise mit äquimolekularen Mengen der Ausgangsmaterialien durchgeführt, gelegentlich wird auch ein Überschuss (bis zu 110%) an Alkin (VI) verwendet. Als Lösungsmittel kommen in Frage: Alkohole (z.B. Methanol, Ethanol), Ether, Kohlenwasserstoffe (z.B. Benzol, Toluol), chlorierte Kohlenwasserstoffe, DMSO, DMF und Pyridin. Die Reaktionstemperatur liegt zwischen 20°C und 180°C, vorzugsweise zwischen 35°C und 120°C.

Es ist zweckmässig, unter Sauerstoffausschluss zu arbeiten.

Stoffe der Formel I, bei denen $R^3$ für Alkyl (gegebenenfalls substituiertes) steht, lassen sich aus den entsprechenden Verbindungen, bei denen $R^3$ für Wasserstoff steht, nach den üblichen Alkylierungsmethoden herstellen, z.B. durch Umsetzung mit $CH_3J$, $C_2H_5Br$ oder ω-Bromacetophenon.

Der Nachweis der lipoxygenasehemmenden Eigenschaften der erfindungsgemässen Verbindungen erfolgte analog der Methode von Bailey et al., Journal of Biol. Chemistry 255, 5996, (1980) und nach Blackwell und Flower, Prostaglandins 16, 417 (1978). Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschliessend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8, 10-heptadekatriensäure (HHT) bzw. das Lipoxygenaseprodukt 12-Hydroxy-5,8,11,14-eikosatetraensäure (HETE) unter dem Einfluss der Inhibitoren stellt ein Mass für die Hemmung der Enzyme dar.

Die Lipoxygenasehemmung der Verbindung gemäss Formel I lässt sich an der Hemmung der HETE-Synthese messen. Es zeigt sich, dass die Synthese von $TXB_2$ und von HHT unbeeinflusst bleibt, während der Arachidonsäureumsatz abnimmt. Wie aus der nachfolgenden Tabelle ersichtlich ist, bewirken Verbindungen gemäss Formel I eine signifikante Hemmung der Plättchenlipoxygenase (HETE-Synthese). (Vergl. Tab. 1)

Analog den vorstehenden Ausführungen lassen sich die lipoxygenasehemmenden Eigenschaften der Verbindungen nach Formel I auch an Leukozyten nachweisen.

Die polymorphkernigen Leukozyten des Menschen und des Kaninchens metabolisieren die Arachidonsäure zu 5-Hydroxy-5,8,11,14-eikosatetraensäure (5-HETE) und Leukotrien $B_4$ (5S, 12R-Dihydroxy-6 cis, 8,10-trans-14 ciseikosatetraensäure). Die Hemmung der Freisetzung von 5-HETE und Leukotrien $B_4$ aus den Leukozyten stellt ein Mass für den lipoxygenasehemmenden Effekt der Verbindungen gemäss Formel I dar. (Vergl. Tab. 1)

Der Test mit den Humanleukozyten wurde nach Borgeat und Samuelsson (j. Biol. Chem. 254; 2643, 1979 und Proc. Natl. Acad. Sci. USA, 76, 2148 1979), mit Kaninchenleukozyten nach Walker und Parish (Inter. Archs. Allergy appl. Immun. 66, 83, 1981) durchgeführt.

Der Nachweis der Prostacyclin-stimulierenden Wirkung erfolgte durch Bestimmung der Freisetzung von Prostacyclin nach einstündiger Inkubation von Kaninchenaortenstreifen mit den erfindungsgemässen Verbindungen (analog nach Moncada et al., Lancet 1977, I, 18) und anschliessender radioimmunologischer Bestimmung des stabilen Prostacyclinmetaboliten 6-Keto-PGF 1 (B.M. Peskar et al., FEBS Letters 121, 25, 1980).

Die Verbindungen gemäss Formel I sind auch in vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66, 81 (1981)).

Die Wirkstoffe gemäss Formel I können in bekannter Weise in die üblichen Formulierungen, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel überführt werden. Hierbei sollte die therapeutisch wirksame Verbindung jeweils in einer Konzentration von etwa 0,5 bis 90 Gew.% der Gesamtmischung vorhanden sein, d.h. in Mengen, die ausreichend sind, um den angestrebten Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuss-(Sesamöl), Alkohole (z.B. Ethylalkohol, Glycerin), Glykole (z.B. Propylenglykol, Polyethylenglykol), N-Alkylpyrrolidone, feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere cutan. Im Falle der oralen Anwendung können Tabletten selbstverständlich ausser den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den obengenannten Hilfsstoffen mit verschiedenen Geschmacksaufbesserern oder gefärbten bzw. farbgebenden Stoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verarbreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund der Art und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Die obigen Ausführungen gelten für die Applikation sowohl in der Human- als auch in der Tiermedizin in sinngemäss gleicher Weise.

Die folgenden Beispiele sollen die Erfindung näher erläutern (siehe auch Tabelle 1):

Beispiel 1

3-Methyl-4H-1,4-benzothiazin-2-carbonsäure-(2-cyanethyl)-ester

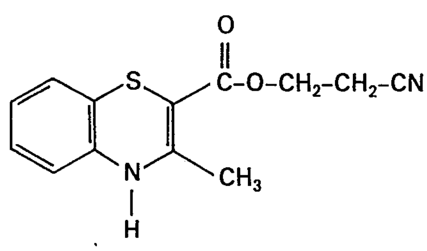

10 g (0,08 Mol) 2-Aminothiophenol und 12,4 g (0,08 Mol) Acetessigsäure-(2-cyanethyl)-ester wurden in 40 ml Dimethylsulfoxid gelöst. Das Gemisch wurde 2 h zum Sieden erhitzt und nach dem Erkalten mit 250 ml Wasser versetzt. Es bildete sich ein Niederschlag, der abgesaugt und aus Isopropanol umkristallisiert wurde.

Fp. 152—154°C

Ausbeute: 8,2 g (39% d.Th.).

In gleicher Weise liessen sich folgende Präparate herstellen:

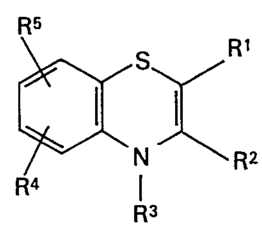

| | $R^1$ | $R^2$ | $R^3$ | $R^4$ | $R^5$ | Fp. |
|---|---|---|---|---|---|---|
| 2 | $-CO_2CH_3$ | $-CH_3$ | H | H | H | 150°C |
| 3 | $-CO_2CH_3$ | $-CH_3$ | $-CH_3$ | H | H | 118°C |
| 4 | $-CO_2C_2H_5$ | $-CH_2-CH_3$ | H | H | H | 122°C |
| 5 | $-CO_2$ 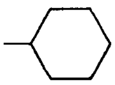 | $-CH_3$ | H | H | H | 161°C |
| 6 | $-CO_2-(CH_2)_2-CN$ | $-CH_3$ | H | H | H | 154°C |
| 7 | $-CO_2C_2H_5$ | $-CH_2-OCH_3$ | H | H | H | |
| 8 | $-CO_2-(CH_2)_2-SCH_3$ | $-CH_3$ | H | H | H | 132°C |
| 9 | $-CO_2-C_4H_9(n)$ | $-CH_3$ | H | H | H | 86°C |
| 10 | $-CO_2-(CH_2)_2-OCH_3$ | $-CH_3$ | H | H | H | 108°C |
| 11 | $-CO-CH_3$ | $-CH_3$ | H | H | H | 196°C |
| 12 | $-CO_2-(CH_2)_2-Ph$ | $-CH_3$ | H | H | H | 136°C |
| 13 | $-CO_2-CH_2-Ph$ | $-CH_3$ | H | H | H | 155°C |
| 14 | H | 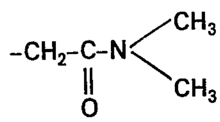 | H | H | H | 163°C |
| 15 | 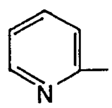 | $-CH_3$ | H | H | H | |
| 15 | -CO-Ph | H | $-CH_2-Ph$ | H | H | 171°C |
| 16 | -CO-Ph | H | $-CH_3$ | H | H | 161°C |
| 17 | $-CO_2CH_3$ | $-CH_3$ | H | $7-CH_3$ | H | |
| 18 | $-CO_2-C_2H_5$ | $-CH_3$ | H | 7-Cl | H | |
| 19 | $-CO-CH_3$ | $-CH_3$ | H | $7-OCH_3$ | H | |
| 20 | $-CO_2-(CH_2)_2-CN$ | $-CH_3$ | H | $6-CH_3$ | H | |
| 21 | $-CO_2-(CH_2)_2-Ph$ | $-CH_3$ | H | 6-Cl | H | |
| 22 | $-CO_2-(CH_2)_2-CN$ | $-CH_3$ | H | $6-CF_3$ | H | |
| 23 | 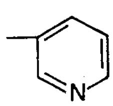 | $-CH_3$ | H | H | H | |
| 24 | 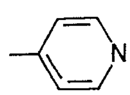 | $-CH_3$ | H | H | H | |
| 25 | 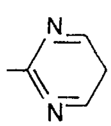 | $-CH_3$ | H | H | H | |
| 26 | $-CO_2-CH_2-CH_2-SCH_3$ | $-CH_3$ | H | 7-Cl | H | 123°C |

Beispiel 27

3-Benzyl-4H-1,4-benzothiazin-2-carbonsäure-ethylester

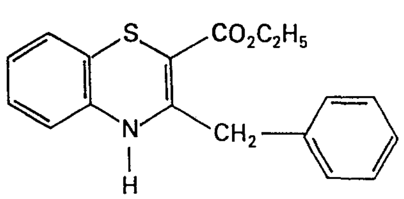

Zu einer siedenden Lösung von 24,8 g (0,1 Mol) 2,2'-Dithiodianilin und 100 mg p-Toluolsulfonsäure in 300 ml Toluol wurde unter Stickstoff eine Lösung von 20,6 g (0,1 Mol) 4-Phenylacetessigsäureethylester in 100 ml Toluol getropft. Das Gemisch wurde 5 Stunden am Wasserabscheider gekocht. Nach dem Erkalten wurde mit Sodalösung gewaschen, die organische Phase abgetrennt, mit $Na_2SO_4$ getrocknet und eingeengt.

Der Rückstand wurde chromatographisch

(Kieselgel 0,063–0,2) aufgetrennt. Es wurden 4,8 g vom Fp. 140–143°C erhalten.

In gleicher Weise (wie Beispiel 27) liessen sich folgende Präparate herstellen:

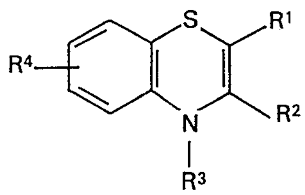

| Beispiel | $R^1$ | $R^2$ | $R^3$ | $R^4$ | Fp. |
|---|---|---|---|---|---|
| 28 | $-CO_2-(CH_2)_2-CN$ | $CH_3$ | H | $7-OCH_3$ | 168°C |
| 29 | $-CO_2-(CH_2)_2-SCH_3$ | $CH_3$ | H | $7-CH_3$ | 154°C |

Beispiel 30

7-Ethoxy-4H-1,4-benzothiazin-2-carbonsäure-ethylester

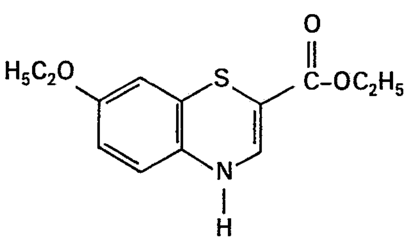

Eine Lösung von 5,05 g 4,4'-Diethoxy-2,2'-Dithio-dianilin und 2,94 g Propiolsäureethylester in 7,5 ml Dimethylsulfoxid wurde 1,5 Std. unter Stickstoff auf 130°C erhitzt. Nach dem Erkalten wurde das Gemisch mit Eiswasser versetzt. Es bildete sich ein Niederschlag, der abgesaugt und in Essigester gelöst wurde. Diese Lösung wurde zweimal mit Wasser gewaschen, mit $Na_2SO_4$ getrocknet und eingeengt. Der Rückstand wurde aus Methanol umkristallisiert.

Fp. 172°C

Ausbeute: 2,3 g

In gleicher Weise (wie Beispiel 30) liessen sich folgende Präparate herstellen:

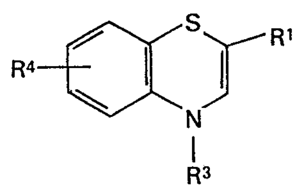

| Beispiel | $R^1$ | $R^3$ | $R^4$ | Fp. |
|---|---|---|---|---|
| 31 | $-CO_2-C_2H_5$ | H | H | 142°C |
| 32 | $-CO_2-C_2H_5$ | H | $7-OCH_3$ | 133°C |

Beispiel 33

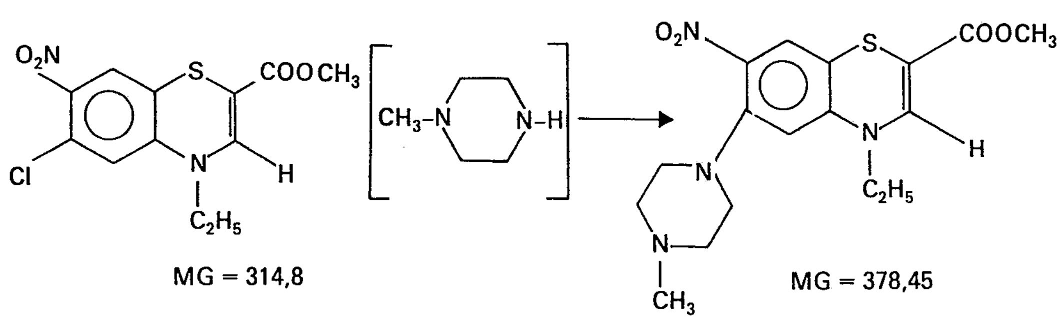

MG = 314,8

MG = 378,45

3,15 g (0,01 Mol)

3,0 g (0,03 Mol) N-Methylpiperazin

50 ml DMSO

Substanzen zusammengegeben und 1,5 Std. bei 50°C laufen lassen. DMSO von der Ölpumpe abdest., Rückstand mit $H_2O/CH_2Cl_2$ extrahiert, org. Phase abgetrennt und eingeengt, Rückstand aus Aceton umkrist.

Ausbeute: 1,4 g

Fp.: 156–159°C.

Beispiel 34

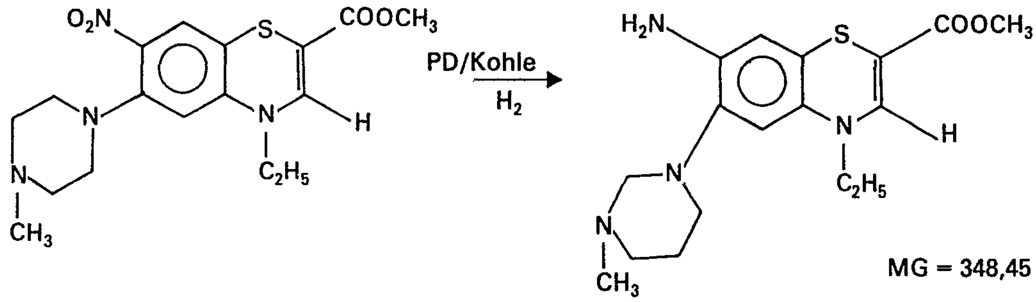


MG = 348,45

8,8 g (0,023 Mol)
250 ml als Methanol
880 mg Pd/Kohle
Insgesamt 16 Std. bei RT $H_2$ eingeleitet, Kontakt abgesaugt ML eingeengt, Rückstand aus Aceton/Ether umkrist.
Ausbeute: 4,5 g
Fp.: 180–186°C

Beispiel 35

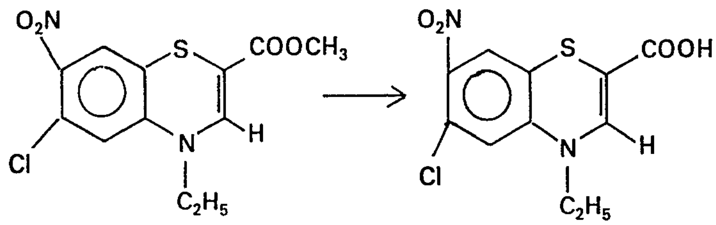

MG 301

2,0 g (0,063 Mol)
20 ml Dioxan
20 ml 6%ige NaOH
Substanzen zusammengeben und 4 Std. auf 50°C erwärmen. Dioxan am Rotowpor abziehen, mit wenig $H_2O$ verdünnt, filtriert. Filtrat mit HCl angesäuert, 24 Std. stehen lassen und abgesaugt.
Ausbeute: 1,5 g
Fp.: 176–180°C

Beispiel 36

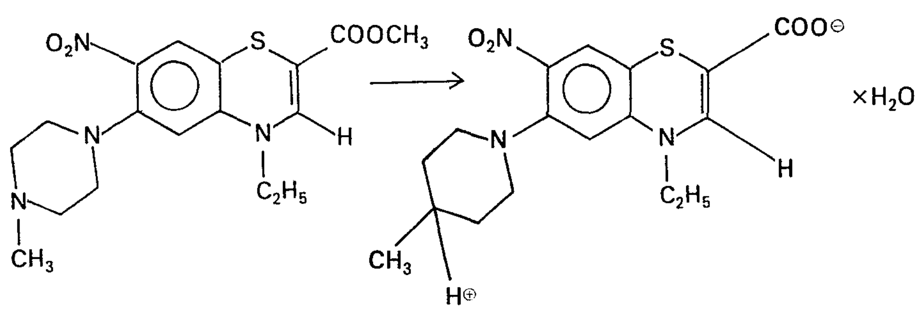


MG = 418,90

2,0 g (0,0053 Mol)
20 ml Dioxan
20 ml 6%ige NaOH
Substanzen zusammengegeben und 3 Std. bei 50°C laufen lassen, Dioxan am Rotowpor abgezogen, Rückstand in wenig $H_2O$ aufgenommen, filtriert. Filtrat mit HCl angesäuert, noch 24 Std. stehen, Kristalle abgesaugt.
Ausbeute: 1,8 g
Fp.: > 250°C

Beispiel 37

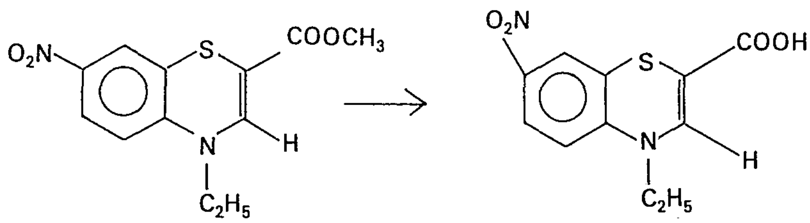

MG = 266,3

1,0 g (0,00357 Mol)
20 ml Dioxan/$H_2O$
0,143 g NaOH
Substanzen zusammengegeben und 3 Std. auf RE erwärmt. Dioxan/$H_2O$ an der Ölpumpe abdest. Rückstand in wenig $H_2O$ aufgenommen, filtriert, mit HCl angesäuert und 24 h stehen lassen. Kristalle abgesaugt.
Ausbeute: 0,4 g
Fp.: 193–195°C

Beispiel 38

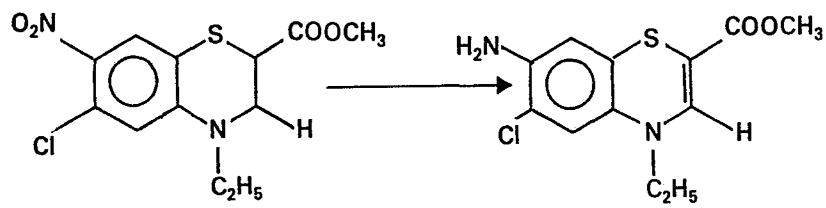

MG = 285

3,15 g (0,01 Mol)
50 ml abs. Dioxan
1,57 g Pd/Kohle
Im 100 ml bei RT hydrieren. Nach 3 Std. RT Kontakt abgesaugt, ML eingeengt, Rückstand in Petrolether aufgeschlämmt und abgesaugt.
Ausbeute: 2,81 g ≅ 98,6% d.Th.
Fp.: 147–150°C

Cyclisierung der offenkettigen Vorstufen mit Kaliumtert.-butylat

Allgemeine Vorschrift:
Man legt 0,01 Mol des σ-(subst.) Phenylsulfinyl-β-ethylaminoacrylsäureesters in 50 ml abs. DMSO vor und gibt bei Raumtemperatur 0,01 Mol frisch sublimiertes Kalium-tert.-butylat portionsweise zu. Nach 8 Stunden bei 100°C wird das DMSO i.Vak. abgezogen, der Rückstand in Wasser/Dichloromethan aufgenommen und die organische Phase abgetrennt. Nach Einengen wird der Rückstand mit Ether kristallisiert.

| Beispiel | dargestellte Verbindungen | Fp (°C) | (Ausbeute) (%) | NMR (Solvens) |
|---|---|---|---|---|
| 39 | 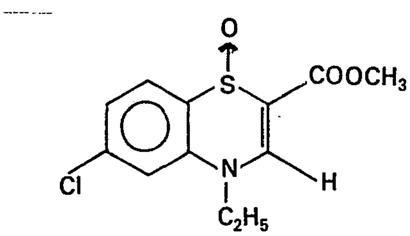 | 172–175 | (90) | ($CDCl_3$) δ=1,5 (3) tr, J=7, δ=3,9 (3) s, δ=4,2 (2) q, J=7, δ=7,45 (1) d, J=9, δ=7,6 (1) dd, J=9, J=3, δ=8,0 (1) d, J=3, δ=8,2 (1) s |
| 40 | 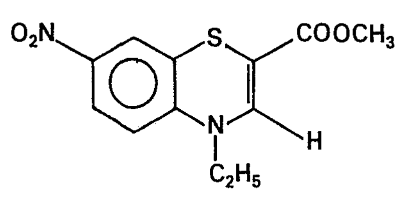 | 162–166 | (90) | ($CDCl_3$): δ=1,3 (3) tr, J=7, δ=3,45 (2)tr, J=7, δ=3,7 (3) s, δ=6,4 (1) d, J=9, δ=6,9 (1) s, δ=7,55 (1) d, J=3, δ=7,75 (1) dd, J=9, J=3 |

| Beispiel | dargestellte Verbindungen | Fp (°C) | (Ausbeute) (%) | NMR (Solvens) |
|---|---|---|---|---|
| 41 | 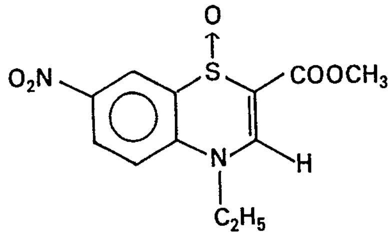 | 185–189 | (85) | ($CDCl_3$): δ=1,55 (4) tr, J=8, δ=3,9 (3) s, δ=4,3 (2) q, J=8, δ=7,65 (1) d, J=9, δ=8,25 (1) s, δ=8,5 (1) dd, J=9, J=3, δ=8,9 (1) d, J=8 |
| 42 | 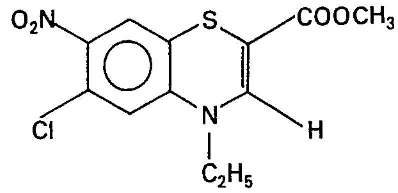 | 163–166 | (76) | ($CDCl_3$): δ=1,3 (3) tr, J=7, δ=3,4 (2) q, J=7, δ=3,75 (3) s, δ=6,4 (1) s, δ=6,9 (1) s, δ=7,4 (1) s |
| 43 | 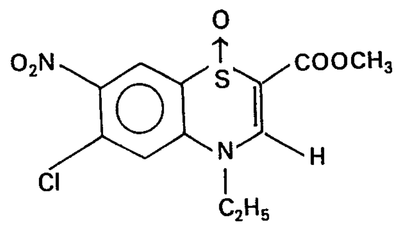 | 157–162 | (85) | ($CDCl_3$): δ=1,55 (3) tr, J=7, δ=3,9 (3) s, δ=4,25 (2) q, J=7, δ=7,6 (1) s, δ=8,0 (1) s, δ=8,7 (1) s |

Tabelle 1 (Wirkungsangaben)

| Substanz Beispiel | | Minimale Konzentration für die Hemmung der Lipoxygenase in Kaninchen-PMN-Leukozyten in g/ml |
|---|---|---|
| 1 | 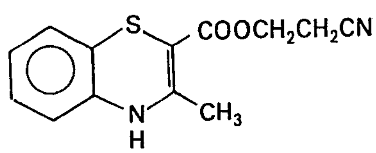 | $5 \times 10^{-7}$–$10^{-6}$ |
| 5 | 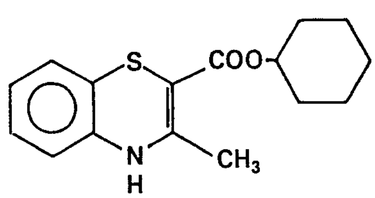 | $5 \times 10^{-7}$–$10^{-6}$ |
| 13 | 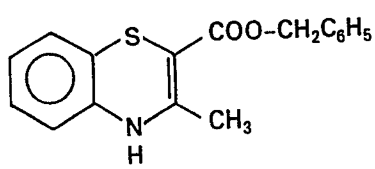 | $5 \times 10^{-7}$–$10^{-6}$ |

Tabelle 1 (Fortsetzung)

| Substanz<br>Beispiel | Minimale Konzentration für die Hemmung der Lipoxygenase in Kaninchen-PMN-Leukozyten in g/ml |
|---|---|
| 2 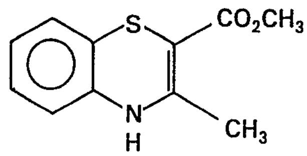 | $5\times10^{-7}$–$10^{-6}$ |
| 4 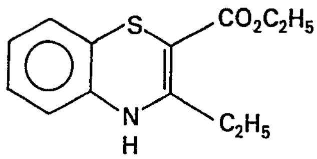 | $5\times10^{-7}$–$10^{-6}$ |
| 8 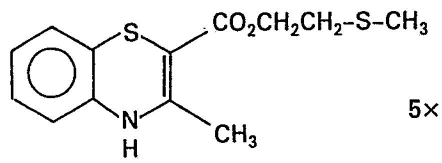 | $5\times10^{-7}$–$10^{-6}$ |
| 12 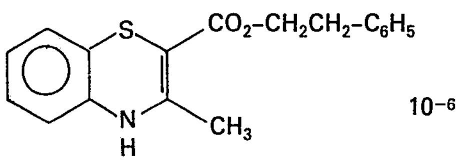 | $10^{-6}$ |
| 9 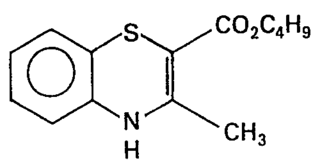 | $10^{-6}$ |
| 11 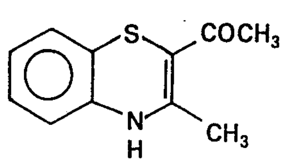 | $10^{-6}$ |
| 26 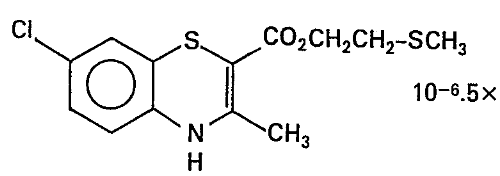 | $10^{-6}$.$5\times10^{-7}$ |
| 28 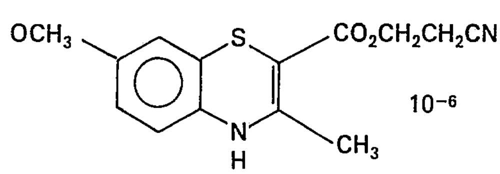 | $10^{-6}$ |

Tabelle 1 (Fortsetzung)

| Substanz<br>Beispiel | | Minimale Konzentration für die Hemmung der Lipoxygenase in Kaninchen-PMN-Leukozyten in g/ml |
|---|---|---|
| 32 | 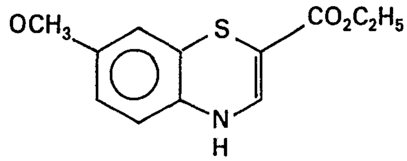 | $10^{-6}$ |
| 37 | 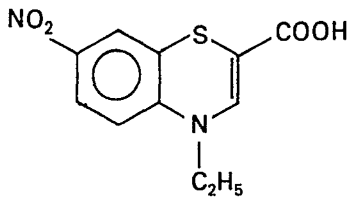 | $5 \times 10^{-6}$ |
| 40 | 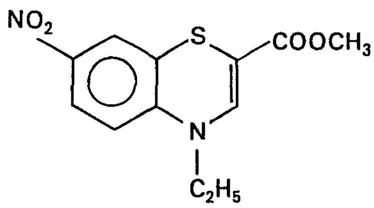 | $5 \times 10^{-6}$ |
| 30 | 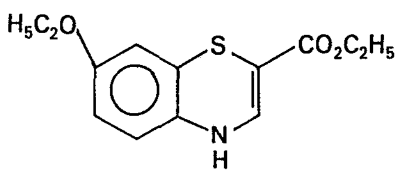 | $10^{-6}$–$5 \times 10^{-7}$ |
| 29 | 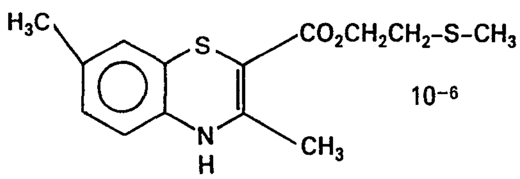 | $10^{-6}$ |

**Patentansprüche**

1. 4H-1,4-Benzothiazine der Formel

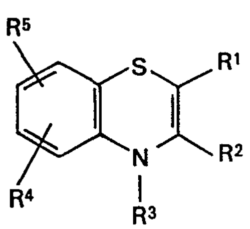

in der

$R^1$ Wasserstoff, Aryl mit 6 bis 20 Kohlenstoffatomen, das gegebenenfalls ein- bis dreimal durch Halogen, Trifluormethyl oder Nitro substituiert sein kann, 2-Thienyl, 3-Thienyl, 2-Furyl, 3-Furyl, 2-Pyridyl, 3-Pyridyl, 4-Pyridyl, 2-Pyrimidyl, 4-Pyrimidyl oder 5-Pyrimidyl, die gegebenenfalls durch Alkyl, Alkoxy, Hydroxy oder Halogen ein- oder mehrfach substituiert sein können, Cyano oder die Gruppe

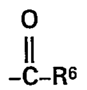

bedeutet, in der

$R^6$ für Alkyl ($C_1$ bis $C_8$), Aryl ($C_6$ bis $C_{20}$), Amino, Pyrrolidino, Piperidino, Morpholino, Cycloalkoxy oder für den Rest

$- O - CH_2 - R^8$

steht, in dem

$R^8$ geradkettiges oder verzweigtes Alkyl mit 1 bis 7 Kohlenstoffatomen, des gegebenenfalls ein- bis dreimal durch Halogen, $C_6$ bis $C_{20}$-Aryl, Hydroxy, Alkoxy, Alkylthio oder Cyano substituiert ist, bedeutet,

$R^2$ Alkyl ($C_1$ bis $C_8$) bedeutet, das gegebenenfalls ein- bis dreimal durch Halogen, Pyrrolidino, Piperidino, Aryl ($C_1$ bis $C_{20}$) oder durch die Gruppe

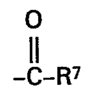

in der

$R^7$ für Aryl ($C_6$ bis $C_{20}$), Hydroxy, Amino Dialkylamino Pyrrolidino, Piperidino oder Morpholino steht, substituiert ist,

$R^3$ Wasserstoff oder eine der unter $R^2$ genannten Gruppen bedeutet, und

$R^4$ und $R^5$ gleich oder verschieden sind, und Wasserstoff, Halogen, Hydroxy, Alkoxy, Nitro, Cyano,

oder eine der unter $R^2$ genannten Gruppen bedeuten, zur therapeutischen Verwendung.

2. 4H-1,4-Benzothiazine zur therapeutischen Verwendung nach Anspruch 1, wobei

$R^1$ Wasserstoff, Phenyl, Naphthyl, gegebenenfalls ein- oder mehrfach durch Fluor und/oder Chlor substituiertes 2-Thienyl, 3-Thienyl, 2-Furyl, 3-Furyl, 2-Pyridyl, 3-Pyridyl, 4-Pyridyl, 2-Pyrimidyl, 4-Pyrimidyl oder 5-Pyrimidyl, ferner Cyano oder die Gruppe

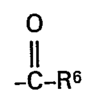

bedeutet, in der

$R^6$ für Alkyl ($C_1$ bis $C_4$), Phenyl, Naphthyl, Amino, Pyrrolidino, Piperidino, Morpholino oder für den Rest

$- O - CH_2 - R^8$

steht in dem

$R^8$ geradkettiges oder verzweigtes Alkyl mit 1 bis 7 Kohlenstoffatomen bedeutet,

$R^2$ Alkyl ($C_1$ bis $C_4$), das gegebenenfalls durch Fluor und/oder Chlor substituiert ist, bedeutet,

$R^3$ Wasserstoff oder eine der unter $R^2$ genannten Gruppen bedeutet, und

$R^4$ und $R^5$ gleich oder verschieden sind und Wasserstoff, Halogen, Hydroxy, Cyano oder eine der unter $R^2$ genannten Gruppen bedeuten.

3. 4H-1,4-Benzothiazine der Formeln

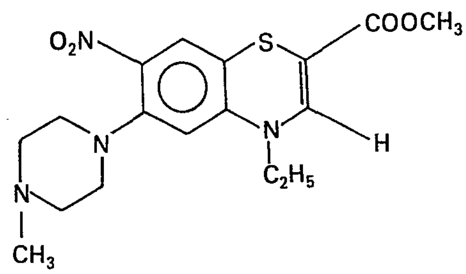

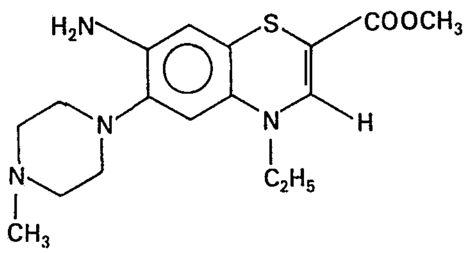

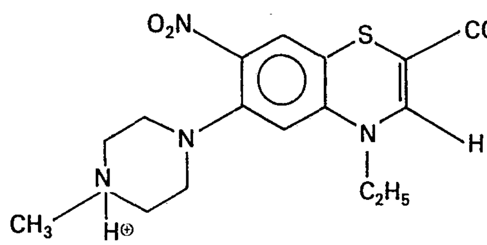

$\times H_2O$

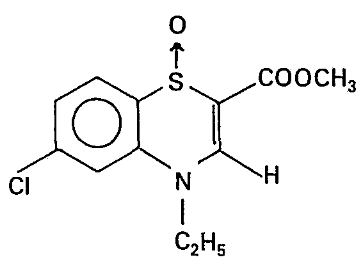

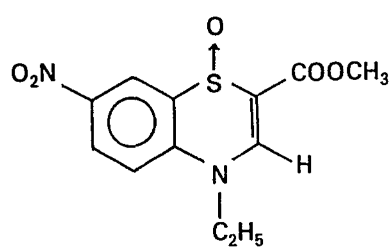

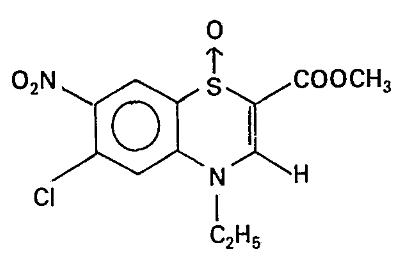

zur therapeutischen Verwendung.

4. Arzneimittel, enthaltend 4H-1,4-Benzothiazine gemäss Anspruch 1.

5. Arzneimittel nach Anspruch 4, enthaltend 4H-1,4-Benzothiazine gemäss Anspruch 2.

6. Arzneimittel, enthaltend 4H-1,4-Benzothiazine gemäss Anspruch 3.

7. Arzneimittel nach den Ansprüchen 3 bis 5, dadurch gekennzeichnet, dass es ein 4H-1,4-Benzothiazin in einer Konzentration von 0,5 bis 90 Gew.% der Gesamtmischung enthält.

8. Verwendung von 4H-1,4-Benzothiazinen gemäss Anspruch 1 zur Herstellung von Arzneimitteln.

9. Verwendung von 4H-1,4-Benzothiazinen gemäss Anspruch 3 zur Herstellung von Arzneimitteln.

10. Verwendung nach den Ansprüchen 8 und 9 zur Herstellung von Lipoxygenasehemmitteln und/oder Antiphlogistika und/oder Antimetastatika.

11. Verfahren zur Herstellung von Arzneimitteln, dadurch gekennzeichnet, dass man 4H-1,4-Benzothiazine der Formel

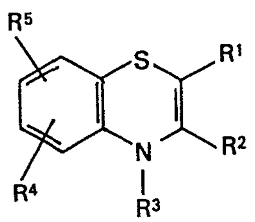

in der

$R^1$ Wasserstoff, Aryl mit 6 bis 20 Kohlenstoffatomen, das gegebenenfalls ein- bis dreimal durch Halogen, Trifluormethyl oder Nitro substituiert sein kann, 2-Thienyl, 3-Thienyl, 2-Furyl, 3-Furyl, 2-Pyridyl, 3-Pyridyl, 4-Pyridyl, 2-Pyrimidyl, 4-Pyrimidyl oder 5-Pyrimidyl, die gegebenenfalls durch Alkyl, Alkoxy, Hydroxy oder Halogen ein- oder mehrfach substituiert sein können, Cyano oder die Gruppe

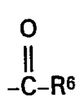

bedeutet, in der

$R^6$ für Alkyl ($C_1$ bis $C_8$), Aryl ($C_6$ bis $C_{20}$), Amino, Pyrrolidino, Piperidino, Morpholino, Cycloalkoxy oder für den Rest

$-O-CH_2-R^8$

steht, in dem

$R^8$ geradkettiges oder verzweigtes Alkyl mit 1 bis 7 Kohlenstoffatomen, das gegebenenfalls ein- bis dreimal durch Halogen, $C_6$ bis $C_{20}$-Aryl, Hydroxy, Alkoxy, Alkylthio oder Cyano substituiert ist, bedeutet,

$R^2$ Alkyl ($C_1$ bis $C_8$) bedeutet, das gegebenenfalls ein- bis dreimal durch Halogen, Pyrrolidino, Piperidino, Aryl ($C_1$ bis $C_{20}$) oder durch die Gruppe

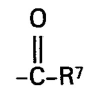

in der

$R^7$ für Aryl ($C_6$ bis $C_{20}$), Hydroxy, Amino, Dialkylamino Pyrrolidino, Piperidino oder Morpholino steht, substituiert ist,

$R^3$ Wasserstoff oder eine der unter $R^2$ genannten Gruppen bedeutet, und

$R^4$ und $R^5$ gleich oder verschieden sind, und Wasserstoff, Halogen, Hydroxy, Alkoxy, Nitro, Cyano, oder eine der unter $R^2$ genannten Gruppen bedeuten, unter Verwendung von üblichen Hilfs- und Trägerstoffen in eine geeignete Applikationsform überführt.

12. Verfahren zur Herstellung von Arzneimitteln, dadurch gekennzeichnet, dass man 4H-1,4-Benzothiazine der Formeln

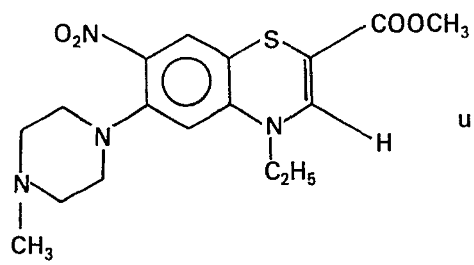

und/oder

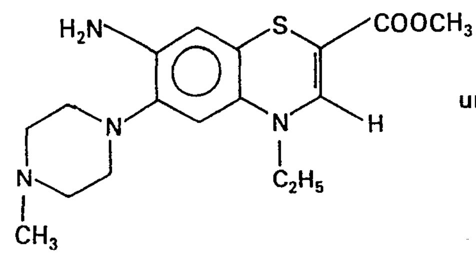

und/oder

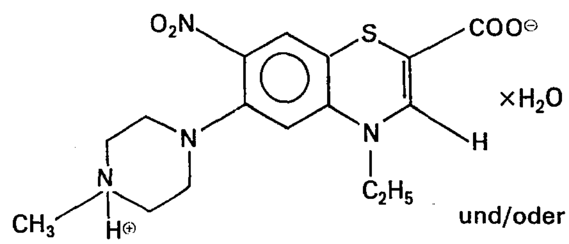

und/oder

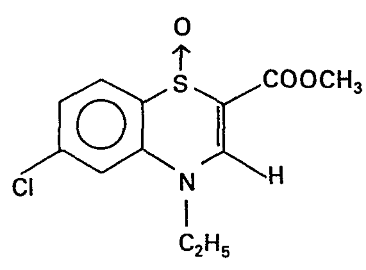

und/oder

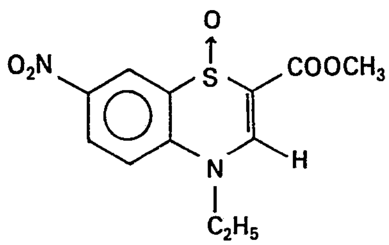

und/oder

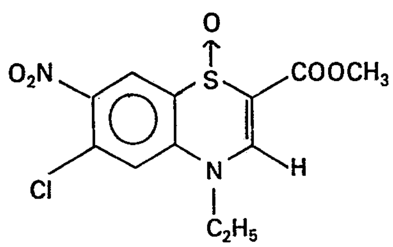

unter Verwendung von üblichen Hilfs- und Trägerstoffen in eine geeignete Applikationsform überführt.

**Claims**

1. 4H-1,4-Benzothiazines of the formula

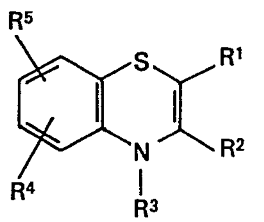

in which

$R^1$ denotes hydrogen, aryl with 6 to 20 carbon atoms, which can optionally be mono- to trisubstituted by halogen, trifluoromethyl or nitro, 2-thienyl, 3-thienyl, 2-furyl, 3-furyl, 2-pyridyl, 3-pyridyl, 4-pyridyl, 2-pyrimidyl, 4-pyrimidyl or 5-pyrimidyl, which can optionally be mono- or polysubstituted by alkyl, alkoxy, hydroxyl or halogen, cyano or the group

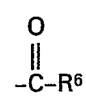

in which

$R^6$ represents alkyl ($C_1$ to $C_8$), aryl ($C_6$ to $C_{20}$), amino, pyrrolidino, piperidino, morpholino, cycloalkoxy or the radical

$-O-CH_2-R^8$

in which

$R^8$ denotes straight-chain or branched alkyl with 1 to 7 carbon atoms, which is optionally mono- to trisubstituted by halogen, $C_6$ to $C_{20}$-aryl, hydroxyl, alkoxy, alkylthio or cyano,

$R^2$ denotes alkyl ($C_1$ to $C_8$), which is optionally mono- to trisubstituted by halogen, pyrrolidino, piperidiono, aryl ($C_1$ to $C_{20}$) or by the group in which

$R^7$ represents aryl ($C_6$ to $C_{20}$), hydroxyl, amino, dialkylamino, pyrrolidino, piperidino or morpholino, $R^3$ denotes hydrogen or one of the groups mentioned under $R^2$, and

$R^4$ and $R^5$ are identical or different and denote hydrogen, halogen, hydroxyl, alkoxy, nitro or cyano, or one of the groups mentioned under $R^2$,

for therapeutic use.

2. 4H-1,4-Benzothiazines for therapeutic use according to Claim 1, wherein

$R^1$ denotes hydrogen, phenyl, naphthyl, 2-thienyl, 3-thienyl, 2-furyl, 3-furyl, 2-pyridyl, 3-pyridyl, 4-pyridyl, 2-pyrimidyl, 4-pyrimidyl or 5-pyrimidyl, which are optionally mono- or polysubstituted by fluorine and or chlorine, and also cyano or the group

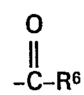

in which

$R^6$ represents alkyl ($C_1$ to $C_4$), phenyl, napthyl, amino, pyrrolidino, piperidino, morpholino or the radical

$-O-CH_2-R^8$

in which

$R^8$ denotes straight-chain or branched alkyl with 1 to 7 carbon atoms,

$R^2$ denotes alkyl ($C_1$ to $C_4$), which is optionally substituted by fluorine and/or chlorine,

$R^3$ denotes hydrogen or one of the groups mentioned under $R^2$, and

$R^4$ and $R^5$ are identical or different and denote hydrogen, halogen, hydroxyl, cyano or one of the groups mentioned under $R^2$.

4H-1,4-Benzothiazines of the formulae

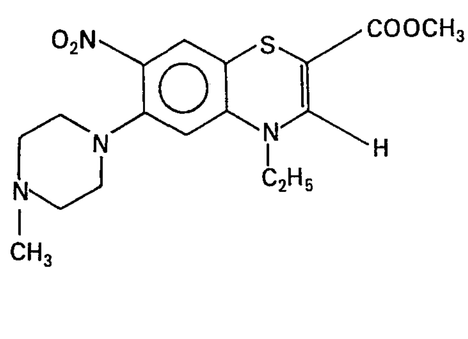

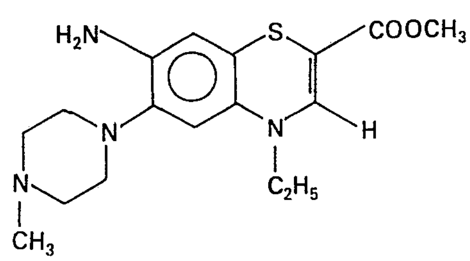

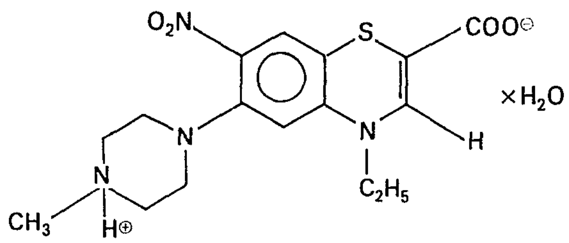

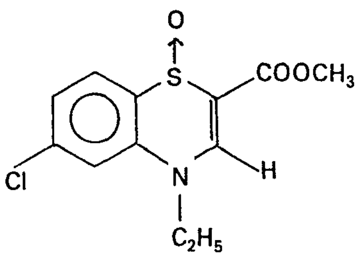

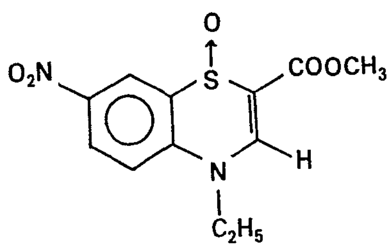

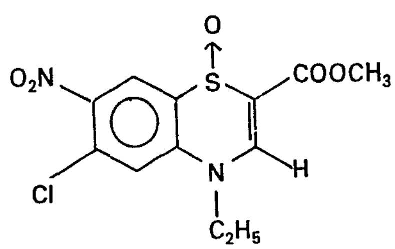

for therapeutic use.

4. Medicaments, containing 4H-1,4-benzothiazines according to Claim 1.

5. Medicaments according to Claim 4, containing 4H-1,4-benzothiazines according to Claim 2.

6. Medicaments, containing 4H-1,4-benzothiazines according to Claim 3.

7. Medicament according to Claims 3 to 5, characterised in that it contains a 4H-1,4-benzothiazine in a concentration of 0.5 to 90% by weight of the total mixture.

8. Use of 4H-1,4-benzothiazines according to Claim 1 for the preparation of medicaments.

9. Use of 4H-1,4-benzothiazines according to Claim 3 for the preparation of medicaments.

10. Use according to Claims 8 and 9 for the preparation of lipoxygenase inhibitors and/or antiphlogistics and/or antimetastatics.

11. Process for the preparation of medicaments, characterised in that 4H-1,4-benzothiazines of the formula

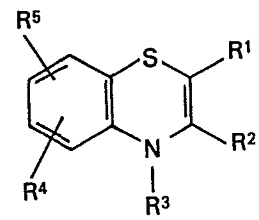

in which

$R^1$ denotes hydrogen, aryl with 6 to 20 carbon atoms, which can optionally be mono- to trisubstituted by halogen, trifluoromethyl or nitro, 2-thienyl, 3-thienyl, 2-furyl, 3-furyl, 2-pyridyl, 3-pyridyl, 4-pyridyl, 2-pyrimidyl, 4-pyrimidyl or 5-pyrimidyl, which can optionally be mono- or polysubstituted by alkyl, alkoxy, hydroxyl or halogen, cyano or the group

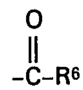

in which

$R^6$ represents alkyl ($C_1$ to $C_8$), aryl ($C_6$ to $C_{20}$), amino, pyrrolidino, piperidino, morpholino, cycloalkoxy or the radical

$$-O-CH_2-R^8$$

in which

$R^8$ denotes straight-chain or branched alkyl with 1 to 7 carbon atoms, which is optionally mono- to trisubstituted by halogen, $C_6$ to $C_{20}$-aryl, hydroxyl, alkoxy, alkylthio or cyano.

$R^2$ denotes alkyl ($C_1$ to $C_8$), which is optionally mono- to trisubstituted by halogen, pyrrolidino, piperidino, aryl ($C_1$ to $C_{20}$) or by the group

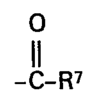

in which

$R^7$ represents aryl ($C_6$ to $C_{20}$), hydroxyl, amino, dialkylamino, pyrrolidino, piperidino or morpholino,

$R^3$ denotes hydrogen or one of the groups mentioned under $R^2$, and

$R^4$ and $R^5$ are identical or different and denotes hydrogen, halogen, hydroxyl, alkoxy, nitro or cyano, or one of the groups mentioned under $R^2$, are converted into a suitable form of administration using customary auxiliaries and excipients.

12. Process for the preparation of medicaments, characterised in that 4H-1,4-benzothiazines of the formulae

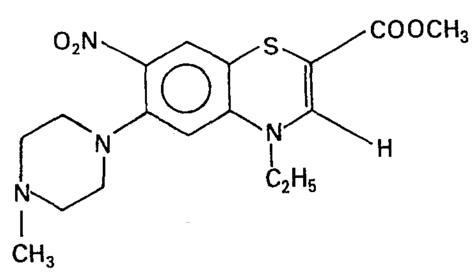

and/or

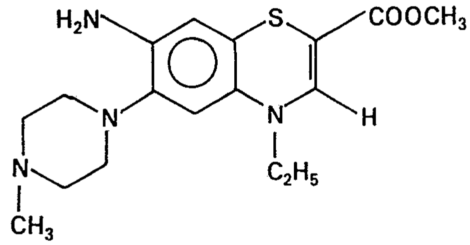

and/or

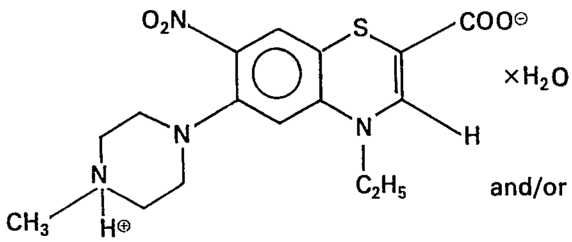

×$H_2O$

and/or

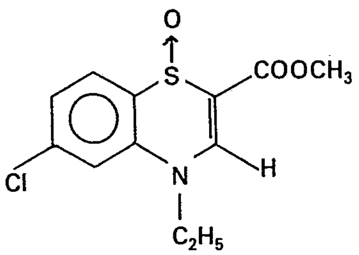

and/or

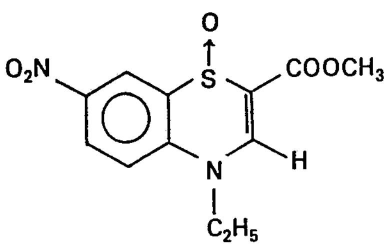

and/or

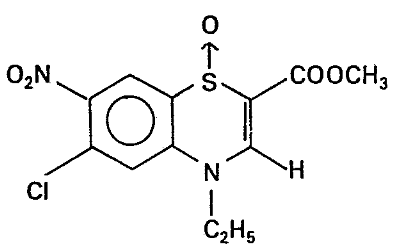

are converted into a suitable form of administration using customary auxiliaries and excipients.

**Revendications**

1. 4H-1,4-benzothiazines de formule

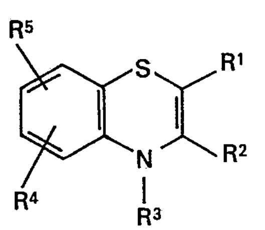

dans laquelle

$R^1$ représente l'hydrogène, un groupe aryle en $C_6$–$C_{20}$ qui peut le cas échéant être mono- a trisubstitué par des halogènes, des groupes trifluorométhyle ou nitro, un groupe 2-thiényle,3-thiényle, 2-furyle, 3-furyle, 2-pyridyle, 3-pyridyle, 4-pyridyle, 2-pyrimidyle, 4-pyrimidyle ou 5-pyrimidyle, qui peuvent le cas échéant être mono- à poly-substitués par des groupes alkyle, alcoxy, hydroxy ou des halogènes, un groupe cyano ou le groupe

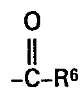

dans lequel

$R^6$ représente un groupe alkyle en $C_1$–$C_8$, aryle en $C_6$–$C_{20}$, amino, pyrrolidino, pipéridino, morpholino, cycloalcoxy ou le groupe

$- O - CH_2 - R^8$

dans lequel

$R^8$ représente un groupe alkyle à chaîne droite ou ramifiée en $C_1$–$C_7$ éventuellement mono- à trisubstitué par des halogènes, des groupes aryles en $C_6$–$C_{20}$, hydroxy, alcoxy, alkylthio ou cyano,

$R^2$ représente un groupe alkyle en $C_1$–$C_8$ éventuellement mono- à trisubstitué par des halogènes, des groupes pyrrolidino, pipéridino, aryle en $C_1$–$C_{20}$ ou par le groupe

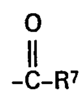

dans lequel

$R^7$ représente un groupe aryle en $C_6$–$C_{20}$, hydroxy, amino, dialkylamino, pyrrolidino, pipéridino ou morpholino,

$R^3$ représente l'hydrogène ou l'un des groupes mentionnées en référence à $R^2$, et

$R^4$ et $R^5$, ayant des significations identiques ou différentes, représentent chacun l'hydrogène, un halogène, un groupe hydroxy, alcoxy, nitro, cyano ou l'un des groupes mentionnés en référence à $R^2$, pour l'utilisation thérapeutique.

2. 4H,1,4-benzothiazines pour l'utilisation thérapeutique selon la revendication 1, dans lesquelles

$R^1$ représente l'hydrogène, un groupe phényle, naphtyle, un groupe 2-thiényle, 3-thiényle, 2-furyle, 3-furyle, 2-pyridyle, 3-pyridyle, 4-pyridyle, 2-pyrimidyle, 4-pyrimidyle ou 5-pyrimidyle éventuellement mono- ou poly-substitué par le fluor et/ou le chlore, ou encore un groupe cyano ou le groupe

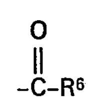

dans lequel

$R^6$ représente un groupe alkyle en $C_1$–$C_4$, phényle, naphtyle, amino, pyrrolidino, pipéridino, morpholino ou le groupe

$$-O-CH_2-R^8$$

dans lequel

$R^8$ représente un groupe alkyle à chaîne droite ou ramifiée en $C_1$–$C_7$,

$R^2$ représente un groupe alkyle en $C_1$–$C_4$ éventuellement substitué par le fluor et/ou le chlore,

$R^3$ représente l'hydrogène ou l'un des groupes mentionnés en référence à $R^2$, et

$R^4$ et $R^5$, ayant des significations identiques ou différentes, représentent chacun l'hydrogène, un halogène, un groupe hydroxy, cyano ou l'un des groupes mentionnés en référence à $R^2$.

3. 4H-1,4-benzothiazines de formules

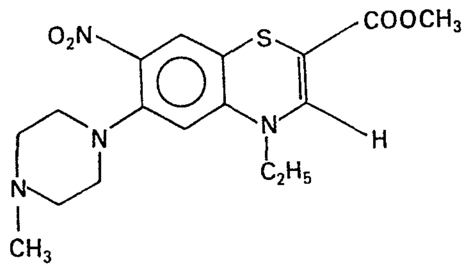

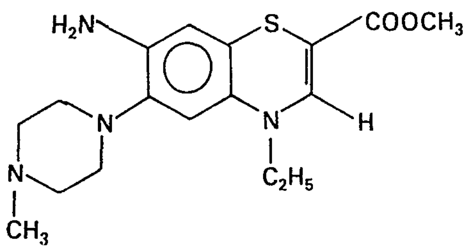

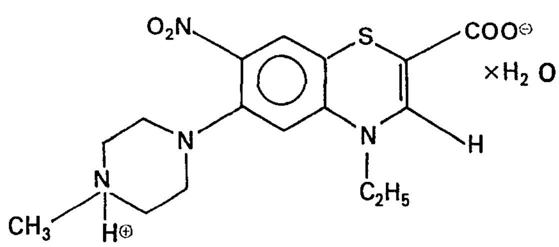

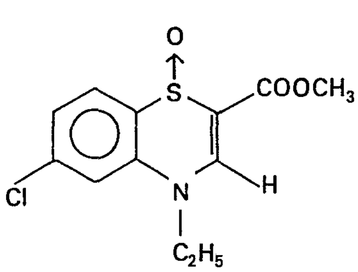

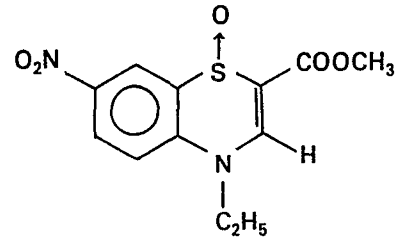

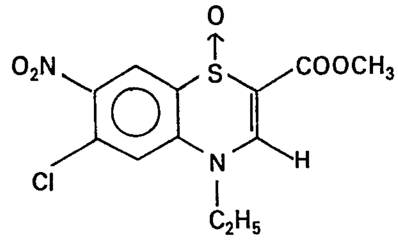

pour l'utilisation thérapeutique.

4. Médicaments contenant des 4H-1,4-benzothiazines selon la revendication 1.

5. Médicaments selon la revendication 4, contenant des 4H-1,4-benzothiazines selon la revendication 2.

6. Médicaments contenant des 4H-1,4-benzothiazines selon la revendication 3.

7. Médicament selon les revendications 3 à 5, caractérisé en ce qu'il contient une 4H-1,4-benzothiazine à une concentration de 0,5 à 90% du poids du mélange total.

8. Utilisation des 4H-1,4-benzothiazines selon la revendication 1, pour la préparation de médicaments.

9. Utilisation des 4H-1,4-benzothiazines selon la revendication 3, pour la préparation de médicaments.

10. Utilisation selon les revendications 8 et 9, pour la préparation d'inhibiteurs de la lipoxygénase et/ou d'antiphlogistiques et/ou d'antimétastatiques.

11. Procédé de préparation de médicaments, caractérisé en ce que l'on met sous une forme d'administration appropriée, par utilisation de produits auxiliaires et véhicules usuels, des 4H-1,4-benzothiazines de formule

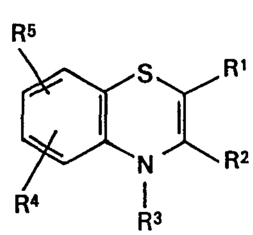

dans laquelle

$R^1$ représente l'hydrogène, un groupe aryle en $C_6$–$C_{20}$ qui peut le cas échéant être mono- à trisubstitué par des halogènes, des groupes trifluorométhyle ou nitro, un groupe 2-thiényle, 3-thiényle, 2-furyle, 3-furyle, 2-pyridyle, 3-pyridyle, 4-pyridyle, 2-pyrimidyle, 4-pyrimidyle ou 5-pyrimidyle qui peuvent le cas échéant être mono- ou

poly-substitués par des groupes alkyles, alcoxy, hydroxy ou des halogènes, un groupe cyano ou le groupe

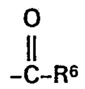

dans lequel

$R^6$ représente un groupe alkyle en $C_1$–$C_8$, aryle en $C_6$–$C_{20}$, amino, pyrrolidino, pipéridino, morpholino, cycloalcoxy ou le groupe

$- O - CH_2 - R^8$

dans lequel

$R^8$ représente un groupe alkyle à chaîne droite ou ramifiée en $C_1$–$C_7$ éventuellement mono- à trisubstitué par des halogènes, des groupes aryles en $C_6$–$C_{20}$, hydroxy, alcoxy, alkylthio ou cyano,

$R^2$ représente un groupe alkyle en $C_1$–$C_8$ éventuellement mono- à trisubstitué par des halogènes, des groupes pyrrolidino, pipéridino, aryles en $C_6$–$C_{20}$ ou par le groupe

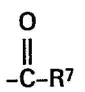

dans lequel

$R^7$ représente un groupe aryle en $C_6$–$C_{20}$, hydroxy, amino, dialkylamino, pyrrolidino, pipéridino ou morpholino,

$R^3$ représente l'hydrogène ou l'un des groupes mentionnés en référence à $R^2$, et

$R^4$ et $R^5$ ayant des significations identiques ou différentes, représentent chacun l'hydrogène, un halogène, un groupe hydroxy, alcoxy, nitro, cyano ou l'un des groupes mentionnés en référence à $R^2$.

12. Procédé de préparation de médicaments, caractérisé en ce que l'on met sous une forme d'administration approprié, par utilisation de produits auxiliaires et véhicules usuels, les 4H-1,4-benzothiazines de formules

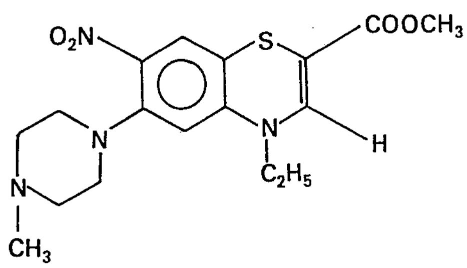

et/ou

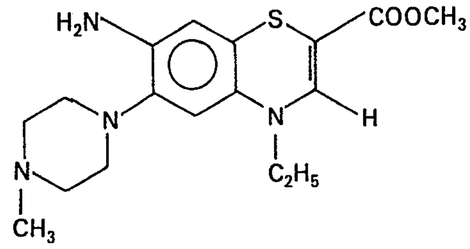

et/ou

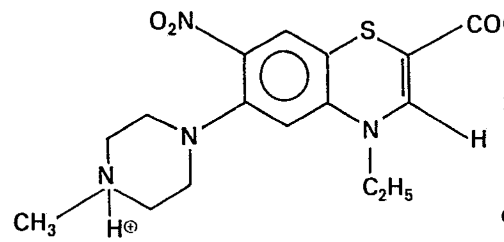

$\times H_2O$

et/ou

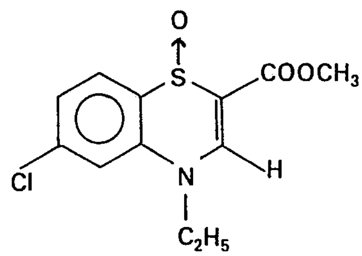

et/ou

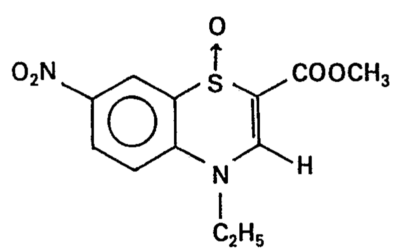

et/ou